# EUROPÄISCHE PATENTSCHRIFT

(11) **EP 4 178 384 B1**
(45) Veröffentlichungstag und Bekanntmachung des Hinweises auf die Patenterteilung: **16.04.2025**
(21) Anmeldenummer: 21737687.0
(22) Anmeldetag: 01.07.2021
(51) Int. Cl.: A24F 42/20, A24F 42/60, A61M 11/02, A61M 15/00, A61M 15/06

(54) **ORALSPENDER FÜR FLÜSSIGKEITEN, INSBESONDERE FÜR NIKOTIN- ODER CANNABISHALTIGE FLÜSSIGKEITEN**
ORAL DISPENSER FOR LIQUIDS, ESPECIALLY FOR LIQUIDS CONTAINING NICOTINE OR CANNABIS
DISTRIBUTEUR ORAL POUR LIQUIDES, EN PARTICULIER POUR DES LIQUIDES CONTENANT DE LA NICOTINE OU DU CANNABIS

(30) Priorität: 07.07.2020 EP 20184586
(43) Veröffentlichungstag der Anmeldung: 17.05.2023
(73) Patentinhaber: Aptar Radolfzell GmbH, 78315 Radolfzell (DE)
(72) Erfinder: GREINER-PERTH, Jürgen, 78244 Gottmadingen (DE)
(74) Vertreter: Witte, Weller und Partner Patentanwälte mbB Stuttgart
(86) Internationale Anmeldenummer: PCT/EP2021/068263
(87) Internationale Veröffentlichungsnummer: WO 2022/008353

(56) Entgegenhaltungen:
- EP-A1- 1 618 803
- WO-A1-2013/089358
- WO-A1-2019/016410

## Beschreibung

### ANWENDUNGSGEBIET UND STAND DER TECHNIK

Die Erfindung betrifft einen Oralspender zum Austrag einer Flüssigkeit in zerstäubter Form, wobei die Erfindung insbesondere einen solchen Spender zum Austrag einer nikotin- oder cannabishaltigen Flüssigkeit betrifft.

Derartige Spender finden insbesondere zur Entwöhnung von Zigarettenkonsum oder als Alternative zu Zigarettenkonsum Verwendung. Sie haben meist eine längliche Form ähnlich einer Zigarette mit einem Mundstück, durch welches verdampfte oder zerstäubte Flüssigkeit durch den Benutzer inhaliert werden kann. Neben nikotin- oder cannabishaltigen Flüssigkeit können auch anderweitige Flüssigkeiten mit Substanzen zur Entwöhnung mit einem erfindungsgemäßen Spender ausgetragen werden. Des Weiteren kann der erfindungsgemäße Spender auch zur Prävention oder Behandlung von akuten oder chronischen Atemwegsbeschwerden oder Atemwegserkrankungen eingesetzt werden.

Ein bekannter Typ zigarettenähnlicher Spender basiert auf einer elektrischen Verdampfung von insbesondere nikotinhaltiger Flüssigkeit. Solche Spender umfassen eine Batterie oder einen Akkumulator, die getauscht oder aufgeladen werden müssen.

Auch bekannt sind Spender, bei denen die nikotinhaltige Flüssigkeit ohne Elektrizität unter Nutzung eines Treibgases zerstäubt wird. Dokument GB 2530980 A zeigt beispielsweise einen solchen Spender. Nachteilig an diesen Spendern ist, dass der Benutzer auch das Treibgas einatmet, wenn er den Spender verwendet. Andere Spender sind aus WO2019/016410A1, WO2013/089358A1 und EP1618803A1 bekannt, wobei das letztgenannte Dokument einen Spender beschreibt , der die Merkmale im Oberbegriff des vorliegenden Anspruchs 1 aufweist.

### AUFGABE UND LÖSUNG

Aufgabe der Erfindung ist es, einen alternativen Spender zur Verfügung zu stellen, mittels dessen Flüssigkeiten, insbesondere nikotinhaltige oder cannabishaltige Flüssigkeiten von einem Benutzer in zerstäubter Form appliziert werden können.

Vorgeschlagen wird hierfür ein Oralspender der eingangs genannten Art, welcher über einen Flüssigkeitsspeicher mit variablem Innenvolumen zur Aufnahme der Flüssigkeit vor dem Austrag verfügt. Als Oralspender im Sinne der Erfindung wird ein Spender angesehen, dessen zerstäubte Flüssigkeit oral appliziert wird, wobei das Ziel der Flüssigkeit beispielsweise der Mundinnenraum selbst, der Rachenraum, der Kehlkopf, die Bronchien oder auch die Lunge sein kann. Vorzugsweise ist ein erfindungsgemäßer Oralspender als Inhalationsspender ausgebildet, bei dem vorgesehen ist, dass der Benutzer während des Austrags der Flüssigkeit einatmet und dadurch die zerstäubte Flüssigkeit in die Atemwege einzieht.

Der Oralspender ist entsprechend dem bevorzugten Anwendungsfeld vorzugsweise mit einer nikotinhaltigen oder einer cannabishaltigen Flüssigkeit befüllt, um hierdurch einen Ersatz für entsprechende Zigaretten darstellen zu können. Allerdings lässt sich ein erfindungsgemäßer Spender auch für anderen Anwendungszwecke nutzen, beispielsweise als Spender für ein Asthma- oder COPD-Medikamente, die üblicherweise mittels so genannter MDI-Spender ausgetragen werden.

Der Oralspender verfügt über eine Federeinrichtung, mittels derer in einem geladenen Zustand der Federeinrichtung die Flüssigkeit im Flüssigkeitsspeicher dauerhaft unter Überdruck gesetzt ist. Diese Federeinrichtung kann eine klassische Feder, beispielsweise eine Schraubenfeder, umfassen. Unter einer Federeinrichtung im Sinne der Anmeldung werden jedoch auch solche Federeinrichtungen verstanden, die anderweitig durch Kompression eines Gases oder Verformung eines elastisch verformbaren Materials in der Lage sind, Energie zu speichern.

Der Flüssigkeitsspeicher ist vorzugsweise zur Erzielung einer geringen Größe des Spenders eher klein. Vorzugsweise weist er ein Volumen von maximal 10 ml auf, insbesondere vorzugsweise von maximal 5 ml oder 3 ml. Unter diesem Volumen ist die Differenz zwischen seinem maximalen Flüssigkeitsinhalt und seinem verbleibenden Flüssigkeitsinhalt, der konstruktionsbedingt nicht mehr ausgetragen werden kann, zu verstehen.

Der durch die Federeinrichtung bewirkte Druck der Flüssigkeit im Flüssigkeitsspeicher dient der Zerstäubung der Flüssigkeit. Zu diesem Zweck weist ein erfindungsgemäßer Oralspender eine Zerstäubungsdüseneinheit auf. Diese verfügt über mindestens eine sehr kleine Düsenöffnung, vorzugsweise über mehrere Düsenöffnungen. Der lichte Querschnitt der mindestens einen Düsenöffnung beträgt maximal 0,002 mm², insbesondere vorzugsweise maximal 0,001 mm² oder maximal 0,0005 mm². Die aufsummierte Fläche der lichten Querschnitte aller Düsenöffnungen beträgt vorzugsweise maximal 0,04 mm². Die Düsenöffnungen sind vorzugweise rund.

Die geringe Düsengröße führt bei ausreichend hohem Druck zu einer feinen Zerstäubung der Flüssigkeit. Vorzugsweise ist vorgesehen, dass die Federeinrichtung derart ausgebildet ist, dass sie im vollständig geladenen Zustand bei vollständig gefülltem Flüssigkeitsspeicher eine Kraft ausübt, die die Flüssigkeit im Flüssigkeitsspeicher unter einen Druck von mindestens 4 bar setzt, vorzugweise von mindestens 6 bar und insbesondere vorzugsweise von mindestens 8 bar. Weiterhin ist vorzugsweise ist vorgesehen, dass die Federeinrichtung auch im Zuge fortschreitender Entleerung des Flüssigkeitsspeichers und damit auch nicht mehr vollständig geladener Federeinrichtung jederzeit einen Druck von mindestens 2 bar, vorzugsweise von mindestens 3 bar in der Flüssigkeit erzeugt. Dies kann dadurch erreicht werden, dass konstruktiv ein vollständiges Entladen der Federeinrichtung verhindert wird, so dass beispielsweise das geringstmögliche Volumen des Flüssigkeitsspeichers erreicht wird, wenn die Federeinrichtung zuvor noch immer einen Druck von mindestens 2 bar, vorzugsweise von mindestens 3 bar, in der Flüssigkeit erzeugen konnte.

Wenn mehrere Düsenöffnungen vorgesehen sind, so sind diese vorzugsweise als Öffnungen in einer gemeinsamen Düsenplatte vorgesehen, welche insbesondere in einem hülsenförmigen Träger aus Kunststoff fixiert sein kann, der im Oralspender fixiert ist, insbesondere in einem Innenbauteil des Oralspenders. Die Düsenplatte kann beispielsweise als Kunststoffplatte, als metallische Düsenplatte oder als Siliziumplatte ausgestaltet sein. Sie kann gewölbt sein, damit die Düsenöffnungen in divergierender Richtung ausgerichtet sind.

Um zu verhindern, dass Partikel in der Flüssigkeit die Düsenöffnungen verstopfen könnten, ist vorzugsweise stromaufwärts der mindestens einen Düsenöffnung ein Flüssigkeitsfilter im Auslasskanal vorgesehen, insbesondere ein Membranfilter.

Der Flüssigkeitsstrom der durch die Zerstäubungsdüseneinheit ausgetragenen Flüssigkeit ist vorzugsweise vergleichsweise gering. Bei einem an der Zerstäubungsdüseneinheit anliegenden Flüssigkeitsdruck von 4 bar und einem vollständig geöffneten Auslassventil beträgt er vorzugsweise zwischen 5 µl/Sekunde und 30 µl/Sekunde, insbesondere vorzugsweise zwischen 10 µl/Sekunde und 20 µl/Sekunde. Dieser geringe Flüssigkeitsstrom hat sich für das durch den Oralspender stattfindende Simulieren einer Zigarette als besonders gut geeignet herausgestellt. Die subjektive Wahrnehmung ähnelt bei solchen Volumenströmen der einer Zigarette.

Der Flüssigkeitsspeicher ist mit der Zerstäubungsdüseneinheit über einen Auslasskanal verbunden. In diesem ist ein Auslassventil vorgesehen, welches einen geöffneten und einen geschlossenen Zustand einnehmen kann, insbesondere indem eine Ventilblende mit einer Ventilblendenöffnung zwischen einer den Auslasskanal unterbrechenden und einer den Auslasskanal freigebenden Stellung verschoben wird. Vorzugsweise ist das Auslassventil derart ausgebildet, dass es auch Zwischenstellungen zulässt, die zu einem reduzierten Volumenstrom führen.

Die von Zerstäubungsdüseneinheit zerstäubte Flüssigkeit gelangt in ein Mundstück, welches bestimmungsgemäß vom Benutzer mit dem Mund umschlossen wird, bevor er das Auslassventil öffnet.

Vorzugsweise ist vorgesehen, dass nicht nur die Zerstäubungsdüseneinheit zur Abgabe in das Mundstück ausgebildet ist, sondern zusätzlich mindestens ein Luftkanal vorgesehen ist, durch den Luft in das Mundstück einströmen kann, wenn der Benutzer am Mundstück zieht. Der Benutzer zieht somit Luft und zerstäubte Flüssigkeit ein. Vorzugsweise sind mehrere Luftkanäle vorgesehen, insbesondere vorzugsweise zwei, drei oder vier, die über den Umfang an einer Innenseite des Mundstücks verteilt sind. Die Mündung in das Mundstück kann durch ein Teilstück des Luftkanals erfolgen, das ringförmig ausgebildet ist. Hierdurch kann erreicht werden, dass die eingesogene Luft in Art eines umlaufenden Luftvorhangs verhindert, dass sich die zerstäubt Flüssigkeit an der Innenseite des Mundstücks niederschlägt.

Der mindestens eine Eingang in den Luftkanal ist vorzugsweise nicht unmittelbar an einer Außenseite des Mundstücks vorgesehen, sondern an einem demgegenüber aufgeweiteten Abschnitt des Gehäuses, so dass die Gefahr verringert wird, dass der Benutzer den Eingang mit den Lippen überdeckt. Der Eingang kann insbesondere radial oder axial entgegen einer Inhalationsrichtung ausgerichtet sein.

Obwohl es bevorzugt ist, dass ein erfindungsgemäßer Oralspender als wiederauffüllbarer Spender ausgebildet ist und zu diesem Zweck eine Nachfüllöffnung aufweist, kann es auch gewünscht sein, einen Oralspender der genannten Art als Einwegspender auszubilden, bei dem ein Nachfüllen von Flüssigkeit in den Flüssigkeitsspeicher nicht vorgesehen ist. Eine Nachfüllöffnung wird in einem solchen Fall nicht benötigt. Es wird jedoch als vorteilhaft angesehen, wenn der Oralspender zur Wiederauffüllung vorgesehen ist. Er verfügt hierzu über eine von der Zerstäubungsdüseneinheit getrennte Nachfüllöffnung, die mit dem Flüssigkeitsspeicher verbunden ist, wobei durch Zuführung von Flüssigkeit durch die Nachfüllöffnung in den Flüssigkeitsspeicher die Federeinrichtung geladen werden kann.

Die genannte Nachfüllöffnung ist vorzugsweise mit einem Einlassventil versehen, welches durch außenseitigen Überdruck und/oder durch mechanischen Druck eines Nachfüllstutzens geöffnet werden kann. Solange kein Nachfüllstutzen eines Nachfüllspenders angesetzt ist, wird das Einlassventil durch innenseitigen Druck in seine Schließstellung gedrückt.

Ein erfindungsgemäßer Oralspender soll vorzugsweise in der Art der Handhabung einer Zigarette gleichen. Er weist daher vorzugsweise eine in einer Haupterstreckungsrichtung ausgerichtete längliche Formgebung auf. Insbesondere vorzugsweise ist seine Außenform vollständig oder weitgehend rotationssymmetrisch, wobei bei weitgehender Rotationssymmetrie das Mundstück und/oder ein Betätigungselement zur Steuerung des Auslassventils Abweichungen von der Rotationssymmetrie darstellen können. Ein Außengehäuse des Oralspenders weist vorzugsweise einen maximalen Durchmesser orthogonal zur Haupterstreckungsrichtung von 16 mm auf, wobei ein über das Außengehäuse hinausragendes bewegliches Betätigungselement für das Auslassventil hierbei keine Berücksichtigung findet. Die Länge in Haupterstreckungsrichtung einschließlich des vorzugsweise am proximalen Ende vorgesehenen Mundstücks beträgt vorzugsweise mindestens 80 mm.

Im Falle der genannten länglichen Formgebung sind die Zerstäubungsdüseneinheit und/oder das Mundstück am proximalen Ende des Oralspenders vorgesehen, was die Funktion des Zigarettenersatzes unterstützt. Die Nachfüllöffnung ist vorzugsweise gegenüberliegend an einem distalen Ende des Oralspenders vorgesehen. Dies ist baulich am einfachsten zu realisieren.

Der Flüssigkeitsspeicher weist ein veränderliches Volumen auf. Dies bedeutet, dass die Entnahme von Flüssigkeit nicht zu einem Unterdruck im Flüssigkeitsspeicher führt und nicht durch einströmende Luft ausgeglichen werden muss. Stattdessen verkleinert sich das durch die Wandungen des Flüssigkeitsspeichers umgebene Volumen im Zuge des Flüssigkeitsaustrags.

Eine bevorzugte Realisierung dessen sieht vor, dass der Flüssigkeitsspeicher zumindest abschnittsweise durch eine formflexible Wandung begrenzt ist. Die Federeinrichtung ist dafür ausgebildet, auf die formflexible Wandung einen in Richtung einer Verkleinerung des Flüssigkeitsspeichers gerichtete Kraft auszuüben. Dabei sind sowohl Gestaltungen denkbar, bei denen die formflexible Wandung selbst die Federeinrichtung darstellt, als auch solche, bei denen die Federeinrichtung separat von der Wandung vorgesehen ist und eine Kraft auf diese ausübt. Auch Kombinationen sind möglich. Eine Gestaltung mit separater Federeinrichtung kann durch eine Gasdruckkammer gebildet sein, die durch die flexible oder einer verschieblichen Wandung vom Flüssigkeitsspeicher getrennt ist. In dieser Gasdruckkammer kann bei minimalem Flüssigkeitsspeichervolumen beispielsweise ein Druck von 3 bar herrschen, der durch das Befüllen des Flüssigkeitsspeichers auf 4 bar ansteigt.

Vorzugsweise ist die formflexible Wandung elastisch verformbar. Sie kann dafür insbesondere aus einem Elastomer gefertigt sein. Eine solche elastische Wandung druckbeaufschlagt in einem durch Verformung gedehnten Zustand die Flüssigkeit im Flüssigkeitsspeicher und bildet daher selbst die Federeinrichtung oder einen Teil der Federeinrichtung.

Eine bevorzugte Ausgestaltung dessen sieht vor, dass die formflexible Wandung als ein den Flüssigkeitsspeicher umgebender elastisch aufweitbarer Schlauch ausgebildet ist. Ist der Flüssigkeitsspeicher befüllt, so ist hierdurch der Schlauch tangential gedehnt.

Besonders bevorzugt ist es, wenn ein vom elastischen Schlauch umgebener Zentraldorn vorgesehen ist. Der Flüssigkeitsspeicher ist bei einer solchen Gestaltung zumindest abschnittsweise ringförmig zwischen einer Außenfläche des Zentraldorns und einer Innenseite des Schlauchs vorgesehen. Der Zentraldorn kann dabei insbesondere gewährleisten, dass auch bei zunehmend entleertem Flüssigkeitsspeicher ein ausreichend hoher Flüssigkeitsdruck besteht, indem er die Volumenverkleinerung des Flüssigkeitsspeichers begrenzt. Sobald der Schlauch außenseitig unmittelbar am Zentraldorn anliegt, ist eine weitere Volumenverringerung nicht möglich, auch wenn der Schlauch noch unter Tangentialspannung steht.

Insbesondere um den Schlauch für eine solche Konfiguration montieren zu können, ist der Zentraldorn zumindest abschnittsweise konisch geformt, vorzugsweise über den überwiegenden Teil seiner Länge. Dies gestattet ein Aufschieben des Schlauches in Richtung des größeren Umfangs des Zentraldorns, wobei während des Aufschiebens die Tangentialspannung verursacht oder vergrößert wird.

Der Zentraldorn kann darüber hinaus mit einem zentralen Flüssigkeitskanal versehen sein, der sich insbesondere in Haupterstreckungsrichtung des Oralspenders erstreckt. Dieser Flüssigkeitskanal kann einen Teil eines Auslasskanals bilden, der den Flüssigkeitsspeicher mit der Zerstäubungsventileinheit verbindet. Alternativ oder zusätzlich kann der Flüssigkeitskanal einen Nachfüllkanal oder einen Teil dessen bilden, der die Nachfüllöffnung mit dem Flüssigkeitsspeicher verbindet. Der Zentraldorn kann einen radialen Durchbruch aufweisen, um den Auslasskanal und/oder den Nachfüllkanal mit dem Ringbereich des Flüssigkeitsspeichers außerhalb des Zentraldorns zu verbinden.

Eine besonders vorteilhafte Gestaltung des Zentraldorns sieht vor, dass dieser zweiteilig ausgebildet ist, insbesondere mit einem inneren Dornbauteil und einem äußeren Dornbauteil. Die zwei Dornbauteile, vorzugsweise das innere und das äußere Dornbauteil, sind dafür ausgebildet durch axiale Verlagerung zueinander im Rahmen der Montage ihren gemeinsamen Außenumfang zu vergrößern. Eine solche Bauweise gestattet eine vorteilhafte Montage, bei der zunächst das eine, insbesondere das äußere, Dornbauteil in den Schlauch eingefügt wird, und bei der anschließend das andere, insbesondere das innere, Dornbauteil an bzw. in das zuvor bereits eingesetzte Dornbauteil eingeschoben wird, so dass sich der gemeinsame Umfang vergrößert und der Schlauch hierdurch tangential geweitet wird.

Bei einer Gestaltung des Spenders mit einem Einlassventil an der Nachfüllöffnung kann vorteilhaft vorgesehen sein, dass mindestens eine Ventilfläche des Einlassventils durch jenes Bauteil gebildet ist, welches einstückig auch den elastischen Schlauch des Flüssigkeitsspeichers bildet.

Eine alternative Variante eines erfindungsgemäßen Oralspenders weist statt einer formflexiblen Wandung eine verschiebliche Wandung auf, die den Flüssigkeitsspeicher zumindest abschnittsweise begrenzt. Diese verschiebliche Wandung ist dabei vorzugsweise in Art eines Kolbens in einen zylindrischen Körper eingesetzt und dichtet im Randbereich gegen diesen ab. Dabei ist die Federeinrichtung dafür ausgebildet, auf die verschiebliche Wandung einen in Richtung einer Verkleinerung des Flüssigkeitsspeichers gerichtete Kraft auszuüben. Die verschliebliche Wandung ist in Richtung der Haupterstreckungsrichtung verschieblich und wird insbesondere vorzugsweise von der Federeinrichtung in Richtung eines proximalen Endes des Oralspenders kraftbeaufschlagt. Die Federeinrichtung wird vorzugsweise durch eine Gasdruckkammer oder eine Schraubenfeder gebildet.

Der Oralspender kann ähnlich dem bereits genannten Zentraldorn einen Führungsdorn aufweisen. Dabei ist der Flüssigkeitsspeicher zumindest abschnittsweise ringförmig zwischen einer Außenfläche dieses Führungsdorns und einer Innenseite einer Außenwand vorgesehen. Der Führungsdorn gewährleistet eine reproduzierbare verkantungsfreie Verlagerung der verschieblichen Wandung. Zudem erlaubt er es, innerhalb des Führungsdorns einen zentralen Flüssigkeitskanal vorzusehen, der sich dadurch durch den Flüssigkeitsspeicher hindurch erstreckt. Dieser kann den Auslasskanal und/oder den Nachfüllkanal darstellen. Insbesondere vorzugsweise erstreckt sich der Flüssigkeitskanal im Führungsdorn als Nachfüllkanal von einem distalen Ende aus bis zu einem zum proximalen Ende weisenden Ende des Flüssigkeitsspeichers und ist dort mittels einer radialen Durchbrechung mit dem Flüssigkeitsspeicher verbunden.

Die Benutzung des Oralspenders sieht vor, dass der Benutzer das Mundstück mit den Lippen umschließt und beim Einatmen das Auslassventil öffnet, so dass die Flüssigkeit durch die Zerstäubungsdüseneinheit zerstäubt wird. Für die Gestaltung des Auslassventils werden verschiedene Bauweisen bevorzugt.

Bei einer besonders einfachen Gestaltung ist das Auslassventil als manuell betätigbares Auslassventil ausgebildet, wird also mit der Hand vom Benutzer bedient. Dabei besonders zweckmäßig ist eine Ausgestaltung mit einem Betätigungsdrücker an einer Außenseite des Oralspenders, vor allem an einer Mantelfläche des Oralspenders, mittels dessen das Auslassventil in einen geschlossenen und einen geöffneten Zustand gebracht werden kann. Ein manuelles Niederdrücken des Betätigungsdrückers, insbesondere gegen die Kraft einer Rückstellfeder, verursacht ein Öffnen des Auslassventils. Der Betätigungsdrücker ist vorzugsweise linearbeweglich.

Möglich ist auch eine Gestaltung mit einem manuell betätigbaren Auslassventil, welches ein drehbares Betätigungselement aufweist, mittels dessen das Auslassventil in einen geschlossenen und einen geöffneten Zustand gebracht werden kann, wobei das Betätigungselement vorzugsweise um eine zur Haupterstreckungsrichtung parallele Drehachse drehbar ist. Beispielsweise könnten ein vorderer und ein hinterer Abschnitt des länglichen Spenders um diese Drehachse gegeneinander verdreht werden, um das als Drehventil ausgebildete Auslassventil zu öffnen.

Eine alternative Gestaltung des Auslassventils sieht vor, dass das Auslassventil als lippenbetätigbares Auslassventil ausgebildet ist. Der Benutzer kann es durch Zusammendrücken seiner Lippen öffnen. Hierzu weist es eine Betätigungsfläche am Mundstück oder im Bereich des Mundstücks auf, welche bei Kraftbeaufschlagung durch die Lippe eines Benutzers die Überführung des Auslassventils aus einem geschlossenen in einen geöffneten Zustand verursacht.

Dies kann beispielsweise realisiert sein, indem das Auslassventil über einen schwenkbeweglichen Betätigungshebel verfügt, der durch Kraftbeaufschlagung der Betätigungsfläche verschwenkbar ist und an dem eine Ventilblende vorgesehen ist, die durch die Schwenkbewegung des Betätigungshebels aus einer Schließstellung in einer Öffnungsstellung überführbar ist.

Eine weitere alternative Gestaltung des Auslassventils ist als atemgesteuertes Auslassventil ausgebildet. Es verfügt hierfür über eine mit dem Mundstück kommunizierende Unterdruckkammer. Wenn der Benutzer am Mundstück saugt, bewirkt dies einen Unterdruck in dieser Kammer. Die Unterdruckkammer ist mit einer verlagerbaren Kammerwandung versehen, welche entweder in sich verformbar ist oder als Ganzes verlagerbar ist, insbesondere vorzugsweise gegen die Kraft einer Rückstellfeder. Die Verlagerung der Kammerwandung wirkt auf ein Ventilelement, beispielweise auf eine Ventilblende im Auslasskanal, so dass der Unterdruck in der Unterdruckkammer das Auslassventil aus einem geschlossenen Zustand in einen geöffneten Zustand überführt.

Das Auslassventil ist vorzugsweise derart ausgebildet, dass die genannten Arten des Schaltens des Ventils, also das manuelle Schalten, das Schalten über die Lippen und das Schalten über das Saugen am Mundstück, jeweils auch ein nicht vollständiges Öffnen des Ventils gestatten. Vorzugsweise bewirken also ein nur leichtes manuelles Betätigen, ein nur leichtes Zusammendrücken der Lippen und ein nur geringes Saugen am Mundstück einen entsprechend verringerten Austrag von zerstäubter Flüssigkeit. Der Benutzer kann somit steuern, wie groß der Strom zerstäubter Flüssigkeit sein soll. Insbesondere realisiert werden kann dies durch eine Ventilblende, die abhängig von der Betätigung verschieden weit aus dem Auslasskanal herausbewegt wird und somit in variablem Maß als limitierende Drossel wirkt.

Die Bauweise des Oralspenders sieht vorzugsweise vor, dass dieser über ein Außengehäuse verfügt, welches auch das Mundstück bildet, und innerhalb dessen vorzugsweise mindestens ein Innenbauteil angeordnet ist. Das Außengehäuse kann bei einer bevorzugten Gestaltung durch zwei Halbschalen gebildet sein, zwischen denen das mindestens eine Innenbauteil vorgesehen ist. Bevorzugt ist eine Gestaltung mit einem Innenbauteil, welches eine Ventilausnehmung aufweist, in der die Ventilblende des Auslassventils beweglich gelagert ist, und/oder welches abschnittsweise den Auslasskanal bildet und/oder welches den Führungsdorn oder den Zentraldorn bildet.

Die vorliegende Offenbarung betrifft gemäß einem zweiten Aspekt weiterhin auch einen Oralspender zum Austrag einer nikotin- oder cannabishaltigen Flüssigkeit, wobei dieser einige oder alle der oben genannten Merkmale einzeln oder in Kombination oder Teilkombination umfassen kann.

Der Oralspender gemäß dem zweiten Aspekt der Erfindung verfügt über einen Flüssigkeitsspeicher zur Aufnahme der nikotin- oder cannabishaltigen Flüssigkeit vor dem Austrag. Bei Auslieferung des Flüssigkeitsspeichers ist dieser vorzugsweise bereits mit der entsprechenden Flüssigkeit befüllt.

Der Flüssigkeitsspeicher ist als Druckspeicher ausgebildet. Dies bedeutet, dass die Flüssigkeit im Flüssigkeitsspeicher unter Druck gelagert ist. Die Druckbeaufschlagung kann beispielsweise durch ein Treibgas oder in oben beschriebener Art und Weise durch eine Federeinrichtung erzielt werden. Der Oralspender verfügt über eine Zerstäubungsdüseneinheit. Diese kann in verschiedener Weise gestaltet sein. Im einfachsten Falle verfügt sie über eine Wirbelkammer, mittels derer die Zerstäubung erzielt wird. Sie kann aber auch in der oben bereits beschriebenen Weise mit einer oder mehreren sehr feinen Düsenöffnungen versehen sein. Die einzelnen Düsenöffnungen weisen dabei vorzugsweise einen lichten Querschnitts von maximal 0,002 mm² auf. Die Gesamtheit der Düsen weist vorzugsweise Summe der lichten Querschnitte von maximal 0,04 mm² auf.

Der Oralspender verfügt über einen Auslasskanal, der den Flüssigkeitsspeicher mit der Zerstäubungsdüseneinheit verbindet. In diesem Auslasskanal ist ein Auslassventil vorgesehen, welches einen geöffneten und einen geschlossenen Zustand einnehmen kann. Ist das Auslassventil geöffnet, so strömt die Flüssigkeit aus dem Druckspeicher in Richtung der Düseneinheit und wird hier durch eine Mundstück des Spenders in zerstäubter Form abgegeben. Das Auslassventil kann beispielsweise in Art einer beweglichen Ventilblende vorgesehen sein, die mit einer Öffnung versehen ist. Wird die Ventilblende und hiermit die Öffnung gegenüber dem Auslasskanal stromaufwärts und stromabwärts verschoben, kann der Durchfluss hierdurch beendet werden. Wird die Öffnung durch Verlagerung der Ventilblende derart verschoben, dass sie mit dem Auslasskanal fluchtet, so kann die Flüssigkeit hindruchströmen.

Gemäß diesem zweiten Aspekt der Erfindung liegt die Besonderheit darin, dass das Auslassventil als mechanisch lippenbetätigbares Auslassventil ausgebildet ist. Es ist hierfür eine Betätigungsfläche am Mundstück oder im Bereich des Mundstücks vorgesehen, welche bei Kraftbeaufschlagung durch die Lippe eines Benutzers in mechanischer Weise die Überführung des Auslassventils aus einem geschlossenen in einen geöffneten Zustand verursacht.

Diese Art der Betätigung ist sehr intuitiv und eignet sich daher besonders gut für einen Nikotin- oder Cannabis-Spender, der recht häufig verwendet wird und bei dem daher gewünscht ist, dass der Austrag nicht mit einer schwierigen Bedienung einhergehen soll, sondern stattdessen möglichst intuitiv möglich sein soll. Es hat sich gezeigt, dass die Lippenbetätigung es dem Benutzer besonders vorteilhaft gestattet, dass Einatmen bzw. Inhalieren zeitlich mit dem mittels der Lippen bewirkten Austrag der Flüssigkeit in zerstäubter Form abzustimmen. Von Vorteil ist es, wenn der Oralspender einen Lufteinlasskanal aufweist, insbesondere am Mundstück, durch den der Benutzer schon vor dem Beginn des Austrags Luft einsaugen kann, um dann während dieses Einsaugens durch die mittels der Lippen aufgebrachten Kraft den Austrag zu beginnen.

Die genannte Betätigungsfläche ist von einem distalen Ende des Mundstücks vorzugsweise weniger als 20 mm entfernt, insbesondere vorzugsweise weniger als 15 mm. Wenn der Benutzer das Mundstück in den Mund nimmt und mit seinen Lippen umschließt, liegt eine seiner Lippen, vorzugsweise die Oberlippe, an dieser Betätigungsfläche an. Die Betätigungsfläche ist insbesondere radial, also in Richtung des durchströmten Mundstücks, niederdrückbar. Der Benutzer kann somit durch Zusammendrücken seiner Lippen das Ventil öffnen. Die Rückkehr in den geschlossenen Ausgangszustand erfolgt vorzugsweise durch Federkraft. Insbesondere vorzugsweise ist das Ventil dafür ausgebildet, auch ein partielles Öffnen zu gestatten. Dies gestattet es dem Benutzer, die Durchflussmenge durch die Aufbringung von Kraft mittels seiner Lippen zu regeln und hierdurch ähnlich dem unterschiedlich starken Saugen an einer Zigarette die Menge des Nikotins oder Cannabis zu bestimmen.

In der oben bereits beschriebenen Weise kann der Flüssigkeitsspeicher mit einem variablen Innenvolumen ausgebildet sein. Er verkleinert sich, wenn Flüssigkeit ausgetragen wird. Ist der Spender als Einweg-Spender ausgebildet, so wird er nach Entleeren des Flüssigkeitsspeichers entsorgt. Er kann jedoch in der oben bereits beschriebenen Weise auch als Nachfüllspender ausgebildet sein. In diesem Falle ist auch hier vorzugsweise vorgesehen, dass der Spender über eine von der Zerstäubungsdüseneinheit getrennte Nachfüllöffnung verfügt, die mit dem Flüssigkeitsspeicher verbunden ist, wobei durch Zuführung von Flüssigkeit durch die Nachfüllöffnung in den Flüssigkeitsspeicher die Federeinrichtung geladen wird.

Die Offenbarung betrifft nicht nur den Oralspender selbst, sondern auch ein Inhalationssystem mit einem solchen Oralspender, welches zusätzlich einen Nachfüllspender umfasst, in dem unter Überdruck stehende Flüssigkeit oder druckbeaufschlagbare Flüssigkeit gelagert ist und der zur Ankopplung an die Nachfüllöffnung des Oralspenders ausgebildet ist. Vorzugsweise ist der Nachfüllspender mit treibmittelfrei unter Druck stehender Flüssigkeit befüllt, die durch ein schaltbares Abgabeventil entnommen werden kann. Dieses Ventil ist vorzugsweise mit einem Nachfüllstutzen versehen, der an die Nachfüllöffnung des Oralspenders angepasst ist und in diese eingeschoben werden kann. Durch das Einschieben werden das Abgabeventil des Nachfüllspenders und das Einlassventil des Oralspenders geöffnet, so dass die Flüssigkeit in den Flüssigkeitsspeicher des Oralspenders einströmen kann und dort die Federeinrichtung wiederaufladen kann.

### KURZBESCHREIBUNG DER ZEICHNUNGEN

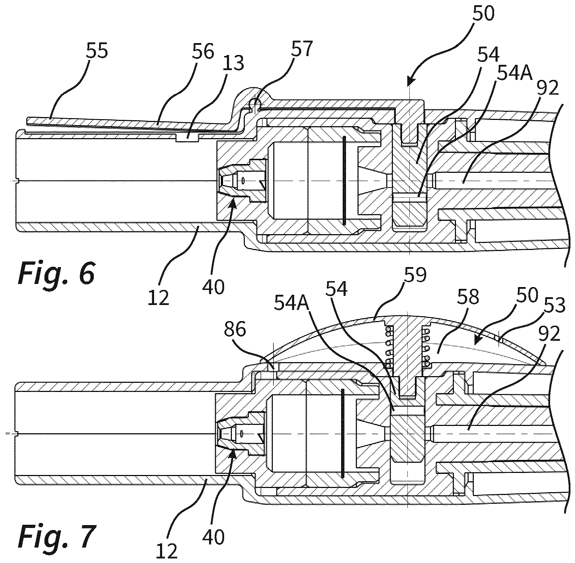

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind. Ein Ausführungsbeispiel in Übereinstimmung mit dem Anspruch 1 ist beispielsweise in Figur 6 gezeigt
Fig. 1 zeigt einen Oralspender gemäß einer ersten vorgeschlagenen Bauform in perspektivischer Ansicht.
Fig. 1A bis 1C zeigen den Oralspender der Fig. 1 in anderen Perspektiven.
Fig. 2A und 2B zeigen den Oralspender der Fig. 1 in zwei geschnittenen Ansichten.
Fig. 2C zeigt in vergrößerter Darstellung die Zerstäubungsdüseneinheit des Oralspenders.
Fig. 3A und 3B verdeutlichen das Aufladen des Oralspenders.
Fig. 3C und 3D verdeutlichen die Verwendung des Oralspenders.
Fig. 4 zeigt einen Oralspender gemäß einer zweiten vorgeschlagenen Bauform in perspektivischer Ansicht.
Fig. 5A und 5B zeigen den Oralspender der Fig. 4 in zwei geschnittenen Ansichten.
Fig 6 und 7 zeigen zwei alternative Konzepte zur Realisierung eines Auslassventils, die bei beiden Gestaltungen eines Oralspenders gemäß Fig. 1 und 4 Verwendung finden können.
Fig. 8A bis 8C zeigen eine alternative Form eines Zentraldorns für den Oralspender gemäß Fig. 1 bis 3D.

### DETAILLIERTE BESCHREIBUNG DER AUSFÜHRUNGSBEISPIELE

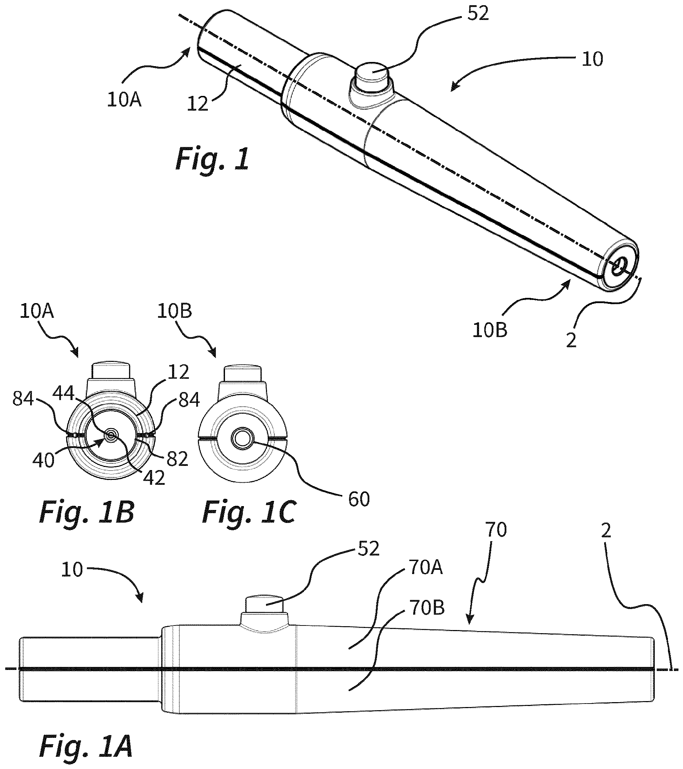

Die Fig. 1 bis 3D zeigen eine erste Ausführungsform eines erfindungsgemäßen Oralspenders.

In Fig. 1 ist der Oralspender 10 in einer perspektivischen Gesamtdarstellung zu sehen. Der Oralspender 10 weist ein in Richtung einer Haupterstreckungsrichtung 2 ausgerichtetes längliches Gehäuse auf, an dessen proximalem Ende 10A ein Mundstück 12 vorgesehen ist. An seiner Mantelfläche ist bei diesem Ausführungsbeispiel ein Betätigungsdrücker 52 vorgesehen, mittels dessen ein weiteres noch erläutertes Auslassventil 50 geöffnet werden kann.

Bezug nehmend auch auf die Fig. 1A bis 1C ist ersichtlich, dass am proximalen Ende in das Mundstück 10 eine Zerstäubungsdüseneinheit 40 gerichtet ist, die über eine Düsenplatte 42 mit einer Vielzahl von Düsenöffnungen 44 verfügt. An einem äußeren Rand des in etwa zylindrischen Mundstücks 12 ist zudem ein Ringkanal 82 vorgesehen, durch den Luft durch das Mundstück 12 eingesogen werden kann. Diese Luft wird zu diesem Zweck durch Einlässe 84 eingesogen.

Am gegenüberliegenden distalen Ende 10B des Oralspenders 10 ist eine Nachfüllöffnung 60 vorgesehen.

Wie in der Fig. 1A zu ersehen ist, weist ein Außengehäuse 70 des Oralspenders 10 zwei Halbschalen 70A, 70B auf. Innerhalb dieser Halbschalen ist eine Mehrzahl von im Weiteren noch beschriebenen Komponenten vorgesehen, die der Lagerung und der gezielten Zerstäubung von Flüssigkeit dienen.

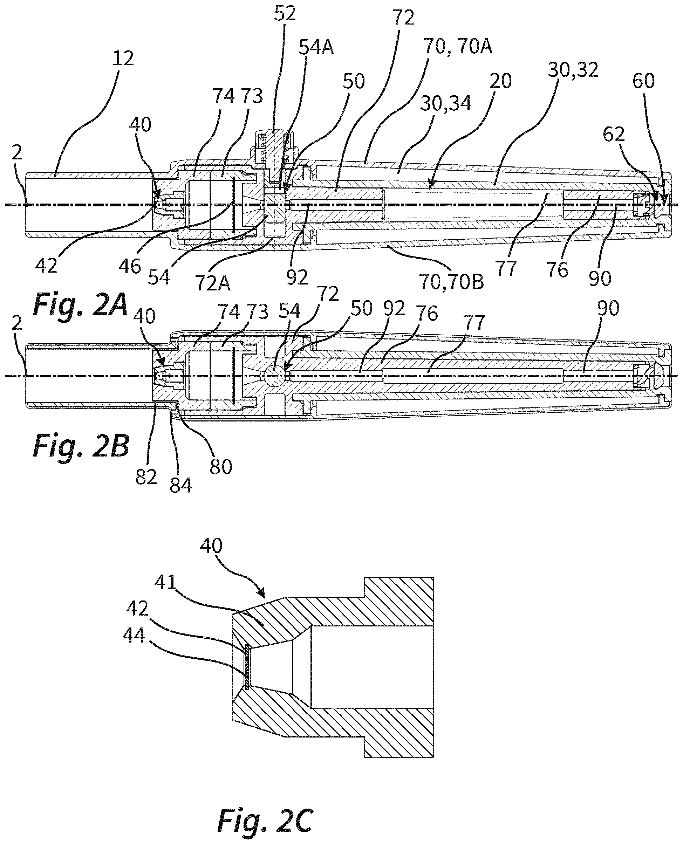

Die Fig. 2A und 2B zeigen den Oralspender 10 in jeweils geschnittener Darstellung, wobei die Schnittebenen um 90° gegeneinander versetzt sind.

Den Figuren ist zu entnehmen, dass innerhalb des Außengehäuses 70 insgesamt drei Innenbauteile 72, 73, 74 vorgesehen sind. Das Innenbauteil 72 bildet an seinem in Fig. 2A rechten Ende einen Zentraldorn 76, der sich bis fast zum distalen Ende 10B des Oralspenders 10 erstreckt. Auf diesen Zentraldorn 76 ist ein schlauchförmiges Bauteil 32 aus einem Elastomer aufgezogen. Ein Zwischenraum zwischen einer Außenseite des Zentraldorns 76 und einer Innenseite dieses Schlauchs 32 bildet ein Flüssigkeitsspeicher 20. In den Darstellungen 2A und 2B ist dieser Flüssigkeitsspeicher nicht befüllt. Der Flüssigkeitsspeicher ist durch radiale Aussparungen 77 mit einem Flüssigkeitskanal 90, 92 verbunden, der sich entlang der Haupterstreckungsrichtung 2 durch den Zentraldorn 76 erstreckt.

Der in Fig. 2A und 2B linke Teil 92 dieses Flüssigkeitskanals bildet einen Teil eines Auslasskanals 92. Ausgehend vom Flüssigkeitsspeicher 20 führt dieser Auslasskanal 92 zum Auslassventil 50. Das Auslassventil 50 weist bei der Gestaltung gemäß den Fig. 1 bis 3D neben dem Betätigungsdrücker 52 eine hieran befestigte Ventilblende 54 mit einer Blendenöffnung 54A auf, die innerhalb einer Ventilausnehmung 72A beweglich ist. Der Betätigungsdrücker 52 ist durch eine Rückstellfeder in die Position der Fig. 2A gedrückt. Wird er gegen die Kraft der Rückstellfeder niedergedrückt, so kann hierdurch die Blendenöffnung54A in eine zum Auslasskanal 92 fluchtende Stellung gebracht werden, so dass die Flüssigkeit durch die Ventilblende 54 hindurch zur rechten Seite gelangt. Rechtsseitig und damit stromabwärts des Auslassventils 50 ist zunächst ein als Membranfilter ausgebildeter Flüssigkeitsfilter 46 vorgesehen, der in das Innenbauteil 73 eingesetzt und vorzugsweise von diesem umspritzt ist. Die durch das Auslassventil 50 hindurchströmende Flüssigkeit wird hier von gegebenenfalls vorhandenen Verunreinigungen befreit, die anderenfalls die Funktion der Zerstäubungsdüseneinheit 40 stören könnten.

Stromabwärts des Membranfilters 46 schließt sich die schon genannte Zerstäubungsdüseneinheit 40 an. Diese weist als Hauptbestandteil eine dünne Düsenplatte, beispielsweise aus Silizium, auf, die von einer Vielzahl von Düsenöffnungen 44 durchdrungen ist. Die Düsenplatte 42 ist in einem hülsenförmigen Kunststoffträger 43 festgelegt, der seinerseits in eine korrespondierende Aussparung des Innenbauteils 74 eingesetzt ist.

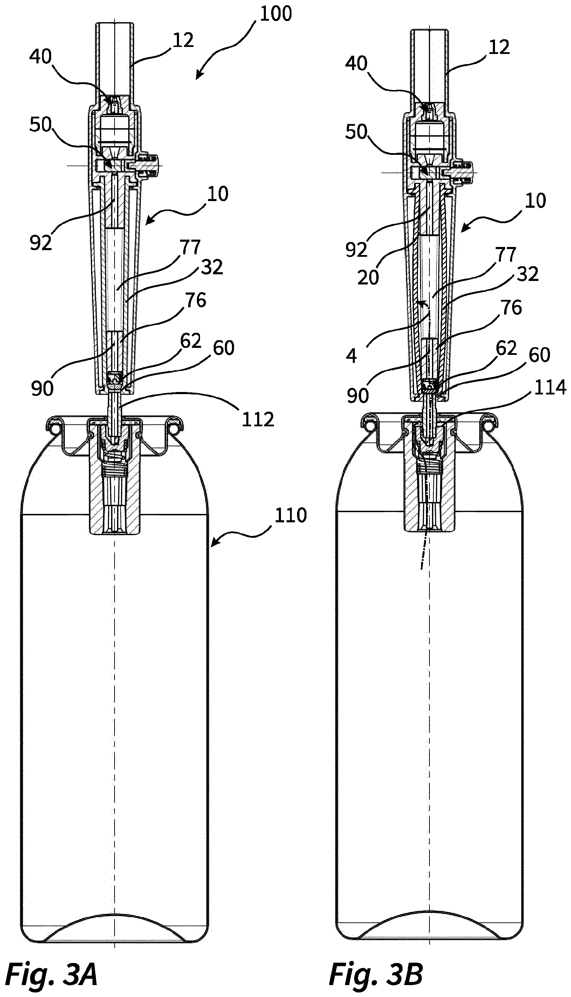

Stromaufwärts des beschriebenen Flüssigkeitsspeichers 20 ist der im Zentraldorn 76 vorgesehene Kanal ein Nachfüllkanal 90, der sich bis zur Nachfüllöffnung 60 erstreckt. Gegenüber einer äußeren Umgebung ist der Flüssigkeitsspeicher 20 jedoch im Normalfall isoliert, da ein als Überdruckventil vorgesehenes Einlassventil 62 vorgesehen ist, welches bei dem üblicherweise im Oralspender 10 herrschenden Überdruck in seine geschlossene Stellung gedrückt wird. Hierfür wird ein Ventilkörper des Einlassventils 62 gegen einen elastisch verformbaren Ringbereich gedrückt, der von dem Bauteil des Schlauchs 32 gebildet wird. Dies wird im Folgenden noch anhand der Fig. 3B erläutert.

Wie anhand der Fig. 2B und auch der Fig. 1C zu ersehen ist und in deren Kontext bereits erwähnt wurde, verfügt der Oralspender 10 über zwei Luftkanäle 80, die jeweils in den bereits genannten Ringkanal 82 münden, der seinerseits in das Mundstück 12 mündet.

In Fig. 2C ist die Zerstäubungsdüseneinheit 40 vergrößert dargestellt. Zu ersehen ist das Hülsenbauteil 41, welches einen Durchgang umgibt, in dem die Düsenplatte 42 mit Düsenöffnungen 44 vorgesehen ist. Die Düsenöffnungen weisen einen lichten Querschnitt von jeweils 100 µm² auf. Beim vorliegenden Ausführungsbeispiel sind insgesamt 25 solche Düsenöffnungen 44 vorgesehen.

Die Fig. 3A und 3B verdeutlichen den Vorgang des Füllens des Oralspenders 10. Zum Befüllen des Oralspenders 10 ist ein Nachfüllspender 110 vorgesehen, der gemeinsam mit dem Oralspender 10 ein Inhalationssystem 100 bildet.

Der Nachfüllspender 110 weist einen Druckspeicher auf, an dessen oberem Ende ein Auslassventil 114 vorgesehen ist, welches mittels eines Ventilstutzens geöffnet werden kann. Der Ventilstutzen bildet einen Nachfüllstutzen 112 zur Einführung in die Nachfüllöffnung 60 des Oralspenders 10. Wird in der in Fig. 3B verdeutlichten Weise der Oralspender 10 nach dem Ansetzen des Nachfüllstutzens 112 an der Nachfüllöffnung 60 niedergedrückt, so werden hierdurch das Auslassventil 114 des Nachfüllspenders 110 und durch Kraftbeaufschlagung mittels des Nachfüllstutzens 112 auch das Einlassventil 62 des Oralspenders 10 geöffnet.

Da im Druckspeicher des Nachfüllspenders 110 ein Druck von 8 bar herrscht, kann durch den Nachfüllstutzen 112 Flüssigkeit in den Nachfüllkanal 90 und bis in den Flüssigkeitsspeicher 20 des Oralspenders gelangen. Der Flüssigkeitspfad 4 verdeutlicht dies.

Dort wird unter dem anliegenden Druck des Nachfüllspenders 110 der Schlauch 32 aufgeweitet und hierbei derart elastisch verformt, dass er nach dem Trennen des Oralspenders 10 vom Nachfüllspender 110 und dem dadurch resultierenden Schließen des Einlassventils 62 einen Druck von etwa 4 bar auf die im Flüssigkeitsspeicher 20 befindliche Flüssigkeit ausübt.

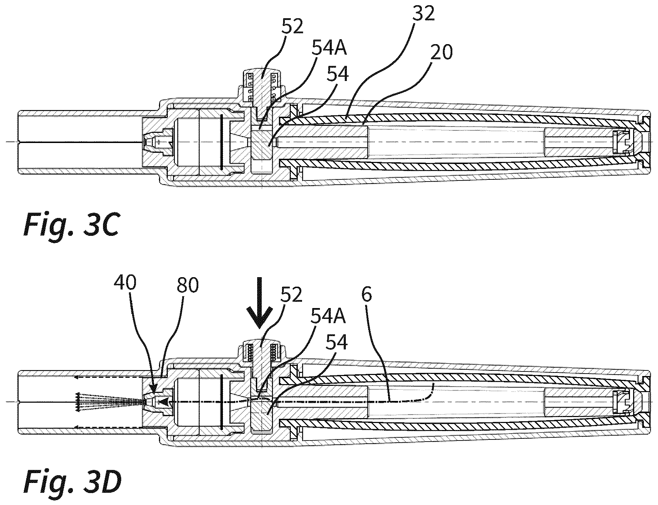

Fig. 3C zeigt den nun geladenen Oralspender 10 mit aufgeweitetem Schlauch 32.

Wird nun in der in der Fig. 3D ersichtlichen Weise das Auslassventil 50 durch Niederdrücken des Betätigungsdrückers 52 geöffnet, so strömt die Flüssigkeit aus dem Flüssigkeitsspeicher 20 entlang des Flüssigkeitspfades 6 durch die Blendenöffnung 54A der Ventilblende 54 zur Zerstäubungsdüseneinheit 40, durch die hindurch die in Fig. 3D durch die gepunkteten Pfeile verdeutlichte Zerstäubung erfolgt. Gleichzeitig saugt der Benutzer am Mundstück 12, wodurch durch die Luftkanäle 80 ein außen liegender Luftmantel im Mundstück 12 erzeugt wird, der gemeinsam mit der zerstäubten Flüssigkeit vom Benutzer inhaliert wird.

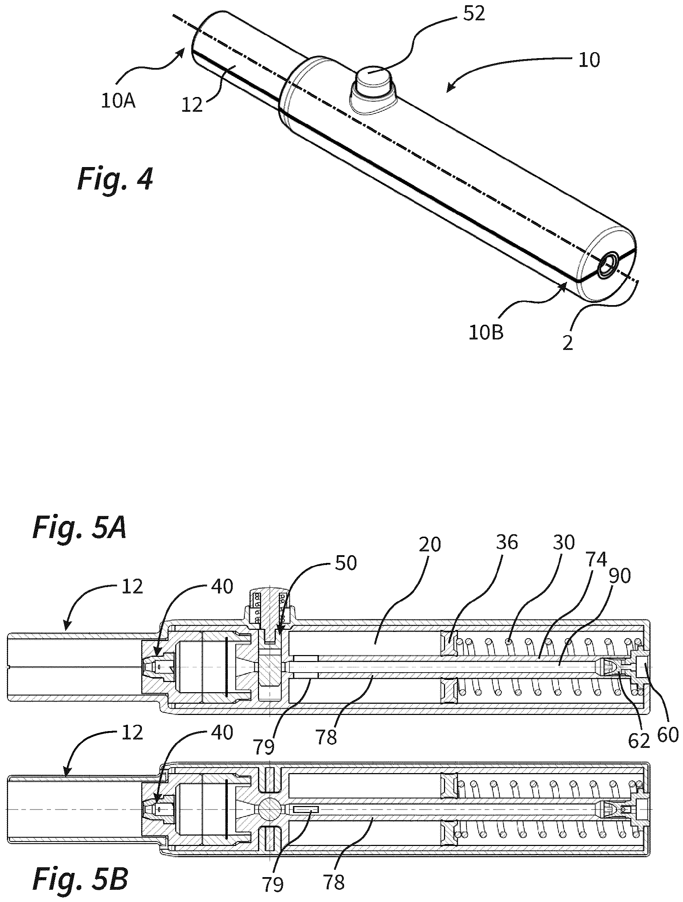

Die Fig. 4 sowie 5A und 5B verdeutlichen eine alternative Bauweise eines Oralspenders 10. Der wesentliche Unterschied zwischen der Bauweise der vorangegangenen Figuren und dieser Gestaltung liegt darin, dass bei dieser Gestaltung der Flüssigkeitsspeicher 20 durch eine verschiebliche Wandung in Art eines Kolbens 36 begrenzt ist. Dieser ist durch einen zentralen Führungsdorn 78 geführt und wird durch eine als Schraubenfeder ausgebildete Federeinrichtung 30 permanent in Richtung des Mundstücks 12 kraftbeaufschlagt. Ist der Flüssigkeitsspeicher 20 gefüllt, so steht die Flüssigkeit aufgrund der Federeinrichtung 30 unter einem Druck, der ausreichend hoch ist, dass in der zuvor schon beschriebenen Art und Weise eine Zerstäubung an der Zerstäubungsdüseneinheit 40 stattfinden kann. Mit fortschreitendem Austrag der Flüssigkeit aus dem Flüssigkeitsspeicher 20 wird der Kolben 36 weiter in Richtung des Mundstücks verlagert.

Wird bei dieser Gestaltung der Nachfüllspender 110 angesetzt, so erfolgt auch hier ein Wiederauffüllen des Flüssigkeitsspeichers 20, während gleichzeitig der Kolben 36 gegen die Kraft der Federeinrichtung 30 weiter ausgelenkt wird. Die Flüssigkeit strömt hierfür von der Nachfüllöffnung 60 durch das hier als Überdruckventil ausgebildete Einlassventil 62 in den Nachfüllkanal 90 und durch radiale Öffnungen 79 im Führungsdorn 78 in den Flüssigkeitsspeicher 20.

Die Fig. 6 und 7 zeigen alternative Ausgestaltungen des Auslassventils, die gleichermaßen bei beiden vorangegangenen Ausgestaltungen des Flüssigkeitsspeichers realisiert sein können.

Bei der Gestaltung gemäß Fig. 6 ist eine Lippenbetätigung des Auslassventils 50 vorgesehen. Hierfür ist ein um die Schwenkachse 57 schwenkbarer Betätigungshebel 56 vorgesehen, dessen proximales Ende eine Betätigungsfläche 55 aufweist, die bestimmungsgemäß durch Zusammendrücken der Lippen des Benutzers niedergedrückt wird. Am gegenüberliegenden Schenkel des Betätigungshebels 56 ist die Ventilblende 54 mit ihrer Blendenöffnung 54A vorgesehen. Wird die Betätigungsfläche 55 nach unten gedrückt, so wird die Blendenöffnung 54A angehoben, so dass sie mit den angrenzenden Teilen des Auslasskanals 92 fluchtet und somit Flüssigkeit aus dem Flüssigkeitsspeicher 20 zur Zerstäubungsdüseneinheit 40 gelangt.

Am Mundstück 12 ist eine Lufteinlassdurchbrechung 13 vorgesehen. Dies gestattet es dem Benutzer, das Mundstück mitsamt der Betätigungsfläche 55 mit den Lippen zu umschließen und dann bereits vor Betätigung der Betätigungsfläche 55 Luft durch die Lufteinlassdurchbrechung 13 hindurch einzusaugen. Währenddessen kann dann durch Zusammendrücken der Lippen und somit Niederdrücken der Betätigungsfläche 55 der Austrag der nikotin- oder cannabishaltigen Flüssigkeit begonnen werden. Durch wohldosierte Kraftbeaufschlagung ist es dem Benutzer dabei auch möglich, die Blendenöffnung 54A nur teilweise mit dem Auslasskanal 92 fluchten zu lassen und dadurch einen nur geringen Austrag von Flüssigkeit zu bewirken. Ähnlich einer Zigarette kann der Benutzer somit entsprechend seinem Wunsch die Abgabe dosiert steuern.

Die Gestaltung gemäß Fig. 7 stellt eine Gestaltung mit einer Atembetätigung des Auslassventils 50 dar. Wird am Mundstück 12 gesogen, so führt dies nicht nur zum Einziehen von Luft durch die bereits beschriebenen Luftkanäle 80, sondern auch dazu, durch einen zusätzlichen Luftkanal 86 Luft aus einer Unterdruckkammer 58 des Auslassventils 50 herausgesogen wird und die flexible Kammerwandung 59 der Unterdruckkammer 58 die Ventilblende 54 daher nach unten drückt. Hierdurch kommt wiederum die Blendenöffnung54A in der Ventilblende 54 in Kommunikation mit angrenzenden Teilen des Auslasskanals 92 und Flüssigkeit strömt zur Zerstäubungsdüseneinheit 40.

Beim Beispiel der Fig. 7 ist die Unterdruckkammer über eine Öffnung 53 mit der Umgebung versehen. Der Strömungswiderstand durch die Öffnung ist größer als der Strömungswiderstand durch den Kanal 86. Dadurch wird der der Unterdruck in der Unterdruckkammer 58 verursacht. Einen zusätzlichen Luftkanal entsprechend den oben beschriebenen Luftkanälen 80 braucht es bei dieser Gestaltung nicht zwingend, denn Luft kann durch die Öffnung 53 und den Luftkanal 86 in das Mundstück gelangen.

Bei alternativen Gestaltungen könnte auch auf die Öffnung 53 verzichtet werden und es könnten stattdessen die beschriebenen Luftkanäle 80 vorgesehen sein. Bei passender Auslegung der jeweiligen Drosselwirkungen der Öffnungen wäre auch damit ein Unterdruck in der Unterdruckkammer 59 erzielbar.

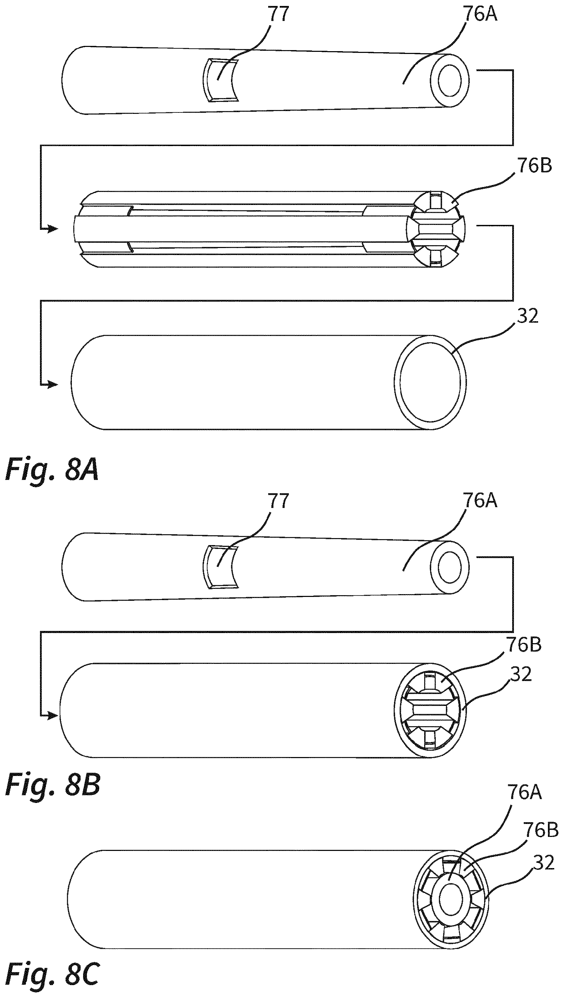

Die Fig. 8A bis 8C zeigen eine besondere Gestaltung des Zentraldorns 76 und die dazugehörige Montage. Der Zentraldorn 76 ist hier in zwei Dornbauteile 76A, 76B untergliedert. Das innere Dornbauteil 76A weist eine leicht konische Form auf. Das äußere Dornbauteil 76B weist eine Mehrzahl von Rippen auf, die umfänglich verteilt angeordnet sind und miteinander durch dünne Verbindungsstege verbunden sind. Die Rippen selbst sind an der Innenseite entsprechend der Konizität des inneren Dornbauteils 76A konisch ausgebildet.

Zu Montage des zweiteiligen Zentraldorns in einem Schlauch 32 ist entsprechend der Figur 8B vorgesehen, dass zunächst das äußere Dornbauteil 76B in den Schlauch 32 eingefügt wird. Dies stellt keine Probleme dar, da im Ausgangszustand der Fig. 8A der Außendurchmesser des äußeren Dornbauteil 76B kleiner als der Innendurchmesser des Schlauchs 32 ist.

Anschließend wird der Verbund aus dem Schlauch 32 und dem äußeren Dornbauteil 76B auf das konische innere Dornbauteil 76A aufgeschoben. Hierdurch weitet sich das äußere Dornbauteil 76B und mit ihm der Schlauch 32, so dass dieser unter die gewünschte Vorspannung gesetzt wird.

Diese Bauweise des Flüssigkeitsspeichers stellte eine bezüglich der Montage verbesserte Version gegenüber der Gestaltung des Zentraldorns 76 der Fig. 1 bis 3D dar.

## Patentansprüche

1. Oralspender (10) zum Austrag einer nikotin- oder cannabishaltigen Flüssigkeit in zerstäubter Form mit den folgenden Merkmalen:
a. der Oralspender (10) verfügt über einen Flüssigkeitsspeicher (20) zur Aufnahme der nikotin- oder cannabishaltigen Flüssigkeit vor dem Austrag, und
b. die Flüssigkeitsspeicher (20) ist als Druckspeicher ausgebildet, in dem die Flüssigkeit unter Überdruck gelagert wird,
c. der Oralspender (10) verfügt über eine Zerstäubungsdüseneinheit (40), und
d. der Oralspender (10) verfügt über einen Auslasskanal (92), der den Flüssigkeitsspeicher (20) mit der Zerstäubungsdüseneinheit (40) verbindet, und
e. im Auslasskanal (92) ist ein Auslassventil (50) vorgesehen, welches einen geöffneten und einen geschlossenen Zustand einnehmen kann, und
f. der Oralspender (10) verfügt über ein Mundstück (12) zur Inhalation der von der Zerstäubungsdüseneinheit (40) zerstäubten Flüssigkeit,
***dadurch gekennzeichnet, dass***
g. das Auslassventil (50) als mechanisch lippenbetätigbares Auslassventil (50) ausgebilde ist und eine Betätigungsfläche (55) am Mundstück (12) oder im Bereich des Mundstücks (12) aufweist, welche bei Kraftbeaufschlagung durch die Lippe eines Benutzers die Überführung des Auslassventils (50) aus einem geschlossenen in einen geöffneten Zustand verursacht.

2. Oralspender (10) nach Anspruch 1 mit dem folgenden weiteren Merkmal:
a. das Auslassventil (50) verfügt über einen schwenkbeweglichen Betätigungshebel (56), der durch Kraftbeaufschlagung der Betätigungsfläche (55) verschwenkbar ist und an dem eine Ventilblende (54) vorgesehen ist, die durch die Schwenkbewegung des Betätigungshebels (56) aus einer Schließstellung in einer Öffnungsstellung überführbar ist.

3. Oralspender (10) nach Anspruch 1 oder 2 mit dem folgenden weiteren Merkmal:
a. der Oralspender (10) weist einen Lufteinlasskanal (13) auf, durch den umgebende Luft eingesogen werden kann,
insbesondere mit einem der folgenden zusätzlichen Merkmale:
b. der Lufteinlasskanal (13) ist stromabwärts der Zerstäubungsdüseneinheit (40) vorgesehen, und/oder
c. der Lufteinlasskanal (13) ist als Durchbrechung am Mundstück (12) vorgesehen.

4. Oralspender nach einem der Ansprüche 1 bis 3 mit dem folgenden weiteren Merkmal:
a. der Flüssigkeitsspeicher (20) weist ein variables Innenvolumen auf.

5. Oralspender nach einem der Ansprüche 1 bis 4 mit dem folgenden weiteren Merkmal:
a. der Oralspender (10) verfügt über eine Federeinrichtung (30), mittels derer in einem geladenen Zustand der Federeinrichtung (30) die Flüssigkeit im Flüssigkeitsspeicher (20) dauerhaft unter Überdruck gesetzt ist.

6. Oralspender nach einem der Ansprüche 1 bis 5 mit dem folgenden weiteren Merkmal:
a. die Zerstäubungsdüseneinheit (40) weist mindestens eine Düsenöffnung (44) mit einem lichten Querschnitt von maximal 0,002 mm² auf, wobei vorzugsweise eine Mehrzahl von Düsenöffnungen (44) vorgesehen sind, deren lichte Querschnitte in Summe maximal 0,04 mm² betragen.

7. Oralspender nach einem der Ansprüche 5 oder 6 mit dem folgenden weiteren Merkmal:
a. der Oralspender (10) verfügt über eine von der Zerstäubungsdüseneinheit (40) getrennte Nachfüllöffnung (60), die mit dem Flüssigkeitsspeicher (20) verbunden ist, wobei durch Zuführung von Flüssigkeit durch die Nachfüllöffnung (60) in den Flüssigkeitsspeicher (20) die Federeinrichtung (30) geladen werden kann.

8. Oralspender nach einem der Ansprüche 1 bis 7 mit dem folgenden weiteren Merkmal:
a. der Flüssigkeitsspeicher (20) ist mit einer nikotin- oder cannabishaltigen Flüssigkeit befüllt.

## Claims

1. Oral dispenser (10) for discharging a nicotine- or cannabis-containing liquid in atomized form, with the following features:
a. the oral dispenser (10) has a liquid reservoir (20) for receiving the nicotine- or cannabis-containing liquid prior to the discharge, and
b. the liquid reservoir (20) is designed as a pressure reservoir in which the liquid is stored under positive pressure,
c. the oral dispenser (10) has an atomization nozzle unit (40), and
d. the oral dispenser (10) has an outlet channel (92) which connects the liquid reservoir (20) to the atomization nozzle unit (40), and
e. an outlet valve (50) which can be in an open state and a closed state is provided in the outlet channel (92), and
f. the oral dispenser (10) has a mouthpiece (12) for inhalation of the liquid atomized by the atomization nozzle unit (40), ***characterized in that***
g. the outlet valve (50) is configured as a mechanically lip-actuatable outlet valve (50) and has on the mouthpiece (12) or in the region of the mouthpiece (12) an actuating surface (55) which, when force is applied by the lip of a user, causes the outlet valve (50) to transfer from a closed state to an open state.

2. Oral dispenser (10) according to Claim 1, with the following further feature:
a. the outlet valve (50) has a pivotingly movable actuating lever (56) which is pivotable by force being applied to the actuating surface (55) and provided on which is a valve orifice element (54) which can be transferred from a closed position to an open position by the pivoting movement of the actuating lever (56).

3. Oral dispenser (10) according to Claim 1 or 2, with the following further feature:
a. the oral dispenser (10) has an air inlet channel (13) through which ambient air can be drawn in,
in particular with one of the following additional features:
b. the air inlet channel (13) is provided downstream of the atomization nozzle unit (40), and/or
c. the air inlet channel (13) is provided as an opening on the mouthpiece (12).

4. Oral dispenser according to one of Claims 1 to 3, with the following further feature:
a. the liquid reservoir (20) has a variable inner volume.

5. Oral dispenser according to one of Claims 1 to 4, with the following further feature:
a. the oral dispenser (10) has a spring mechanism (30) by means of which, in a loaded state of the spring mechanism (30), the liquid in the liquid reservoir (20) is permanently under positive pressure.

6. Oral dispenser according to one of Claims 1 to 5, with the following further feature:
a. the atomization nozzle unit (40) has at least one nozzle opening (44) with a clear cross section of a maximum of 0.002 mm², wherein preferably a plurality of nozzle openings (44) are provided, the clear cross sections of which in total amount to a maximum of 0.04 mm².

7. Oral dispenser according to either of Claims 5 and 6, with the following further feature:
a. the oral dispenser (10) has a refill opening (60) which is separate from the atomization nozzle unit (40) and is connected to the liquid reservoir (20), wherein the spring mechanism (30) can be loaded by liquid being fed through the refill opening (60) into the liquid reservoir (20).

8. Oral dispenser according to one of Claims 1 to 7, with the following further feature:
a. the liquid reservoir (20) is filled with a nicotine- or cannabis-containing liquid.

## Revendications

1. Distributeur oral (10) pour distribuer un liquide contenant de la nicotine ou du cannabis sous forme atomisée, avec les caractéristiques suivantes :
a. le distributeur oral (10) dispose d'un réservoir de liquide (20) pour recevoir le liquide contenant de la nicotine ou du cannabis avant la distribution, et
b. le réservoir de liquide (20) est configuré sous forme de réservoir sous pression, dans lequel le liquide est stocké sous une surpression,
c. le distributeur oral (10) dispose d'une unité de buse d'atomisation (40), et
d. le distributeur oral (10) dispose d'un canal de sortie (92) reliant le réservoir de liquide (20) à l'unité de buse d'atomisation (40), et
e. une soupape de sortie (50) est prévue dans le canal de sortie (92), laquelle peut prendre un état ouvert et un état fermé, et
f. le distributeur oral (10) dispose d'un embout buccal (12) pour inhaler le liquide atomisé par l'unité de buse d'atomisation (40), ***caractérisé en ce que***
g. la soupape de sortie (50) est configurée sous forme de soupape de sortie (50) pouvant être actionnée mécaniquement par une lèvre et présente une surface d'actionnement (55) sur l'embout buccal (12) ou dans la zone de l'embout buccal (12), qui provoque le transfert de la soupape de sortie (50) d'un état fermé à un état ouvert lors de l'application d'une force par la lèvre d'un utilisateur.

2. Distributeur oral (10) selon la revendication 1, avec la caractéristique supplémentaire suivante :
a. la soupape de sortie (50) dispose d'un levier d'actionnement (56) mobile en pivotement, qui peut pivoter par l'application d'une force sur la surface d'actionnement (55) et sur lequel est prévu un diaphragme de soupape (54), qui peut être transféré d'une position de fermeture à une position d'ouverture par le mouvement de pivotement du levier d'actionnement (56).

3. Distributeur oral (10) selon la revendication 1 ou 2, avec la caractéristique supplémentaire suivante :
a. le distributeur oral (10) présente un canal d'entrée d'air (13) à travers lequel l'air ambiant peut être aspiré,
notamment avec l'une quelconque des caractéristiques supplémentaires suivantes :
b. le canal d'entrée d'air (13) est prévu en aval de l'unité de buse d'atomisation (40), et/ou
c. le canal d'entrée d'air (13) est prévu sous forme de percée sur l'embout buccal (12).

4. Distributeur oral selon l'une quelconque des revendications 1 à 3, avec la caractéristique supplémentaire suivante :
a. le réservoir de liquide (20) présente un volume intérieur variable.

5. Distributeur oral selon l'une quelconque des revendications 1 à 4, avec la caractéristique supplémentaire suivante :
a. le distributeur oral (10) dispose d'un dispositif à ressort (30) au moyen duquel, dans un état chargé du dispositif à ressort (30), le liquide dans le réservoir de liquide (20) est mis en permanence en surpression.

6. Distributeur oral selon l'une quelconque des revendications 1 à 5, avec la caractéristique supplémentaire suivante :
a. l'unité de buse d'atomisation (40) présente au moins une ouverture de buse (44) avec une section transversale libre de 0,002 mm² maximum, une pluralité d'ouvertures de buse (44) étant de préférence prévues, dont les sections transversales libres s'élèvent au total à 0,04 mm² maximum.

7. Distributeur oral selon l'une quelconque des revendications 5 ou 6, avec la caractéristique supplémentaire suivante :
a. le distributeur oral (10) dispose d'une ouverture de remplissage (60) séparée de l'unité de buse d'atomisation (40), qui est reliée au réservoir de liquide (20), le dispositif à ressort (30) pouvant être chargé en amenant du liquide à travers l'ouverture de remplissage (60) dans le réservoir de liquide (20).

8. Distributeur oral selon l'une quelconque des revendications 1 à 7, avec la caractéristique supplémentaire suivante :
a. le réservoir de liquide (20) est rempli d'un liquide contenant de la nicotine ou du cannabis.
